# EUROPEAN PATENT SPECIFICATION

(11) **EP 1 922 367 B1**
(45) Date of publication and mention of the grant of the patent: **30.03.2016**
(21) Application number: 06765381.6
(22) Date of filing: 09.08.2006
(51) Int. Cl.: C09C 1/30, C01B 33/18, G06K 9/00

(54) **FINGERPRINT DETECTION USING HYDROPHOBIC SILICA PARTICLES AND METHODS OF MAKING SAME**
FINGERABDRUCKERKENNUNG DURCH VERWENDUNG HYDROPHOBER KIESELSÄUREPARTIKEL UND HERSTELLUNGSVERFAHREN DAFÜR
DETECTION D'EMPREINTES DIGITALES AU MOYEN DE PARTICULES DE SILICE HYDROPHOBES ET PROCEDES DE PRODUCTION DESDITES PARTICULES

(30) Priority: 09.08.2005 GB 0516272; 09.08.2005 GB 0516271; 09.08.2005 US 706439 P; 09.08.2005 US 706438 P; 28.04.2006 GB 0608464; 28.04.2006 US 795599 P; 26.05.2006 GB 0610453; 17.07.2006 US 831204 P
(43) Date of publication of application: 21.05.2008
(73) Proprietor: University of Sunderland, Sunderland SR1 3SD (GB)
(72) Inventor: ROWELL, Frederick John, Chester-le-street Durham DH3 3LS (GB)
(74) Representative: Selby, David Sean
(86) International application number: PCT/GB2006/050233
(87) International publication number: WO 2007/017700

(56) References cited:
- EP-A2- 1 369 907
- WO-A-01/62232
- US-A- 4 176 205
- US-A- 4 837 260
- US-A1- 2002 055 051
- RAO, A. VENKATESWARA ET AL: "Synthesis and Characterization of Hydrophobic Silica Aerogels Using Trimethylethoxysilane as a Co-Precursor" JOURNAL OF SOL-GEL SCIENCE AND TECHNOLOGY , 27(2), 103-109 CODEN: JSGTEC; ISSN: 0928-0707, 2003, XP002404003

## Description

### Field of the invention

The invention relates to a method of synthesising blank and doped hydrophobic silica particles and the use of these particles in, for example, fingerprinting and other applications.

### Background to the invention

The development of latent fingerprints generally involves either the use of a dusting agent that adheres to the "sticky" material deposited on the surface following contact, or a chemical developer that produces a visual colouration due to chemical interaction of the applied developer with chemicals commonly found within the deposited materials on the surface [1]. The size and shape of the powder particles have a large influence on the amount of adhesion they have to the fingerprint, and fine particles tend to adhere better than larger particles, thus resulting in most particles being based on particle sizes of 1-10M [2]. A range of dusting agents are commercially available which are based on a variety of particles, both naturally occurring and synthetic that show affinity for the hydrophobic materials within the deposited print.

With recent innovations in nanotechnology, potential alternative approaches have been studied. Cadmium sulphide and Europium (III) oxide based powders have been used to visualise latent fingerprints [3-5]. However these methods are complex and require specialist technical development and expensive instruments and hence are unsuitable to be used *in situ,* for example at scenes of crime.

Two reports describe the use of combinations of tetraethoxysilane (TEOS) and phenyltriethoxysilane (PTEOS) to produce relatively hydrophobic silica aerogels [3] and the corresponding nanoparticles [4] for use in bioanalysis and biosensor applications. The former report demonstrated that as the proportion of PTEOS increased, the hydrophobicity of the resulting sol gel also increased, whilst the latter report used the particles' hydrophobicity to incorporate the hydrophobic dye, rhodamine 6G into the resulting particles. The nanoparticles were highly fluorescent with the dye being strongly retained within the particles under aqueous conditions. However these particles are synthesised using a multi-step route.

### Summary of the invention

In a first aspect of the invention, there is provided a method for detecting and/or identifying a fingerprint or other hydrophobic mass or deposit, comprising contacting an area where there is or maybe a fingerprint with an agent comprising hydrophobic silica particles, wherein the method comprises applying the agent as a dusting agent or as a suspension which comprises water and a water-miscible solvent to the area.

A novel, single-step synthetic route has now been developed for producing hydrophobic silica particles. This synthetic route is simpler and can be applied to the production of a variety of micro or nanoparticles which optionally incorporate dyes (for example coloured or fluorescent) and magnetic or paramagnetic subparticles into the silica monomer backbone.

This approach overcomes problems arising from direct incorporation of fluorescent or coloured molecules into the particles during their synthesis, or their sol gel equivalents, since the resulting particles or gels generally lose the dyes when the particles are washed. This observation implies that there are only weak interactions between embedded molecules within the crossed linked silicate backbone of the particles' matrix so that dye molecules are not subjected to attractive forces which would retain them within the matrix.

The present invention has also demonstrated the use of these hydrophobic silica micro and nanoparticles as developing agents for latent fingerprints.

Thus, the present disclosure provides a method for preparing hydrophobic silica particles, the method comprising reacting together in a single step a mixture of (1) silane ether monomers, for example, an alkoxysilane and (2) organically substituted silane ether monomers, for example a phenyl modified silane ether, and (3) a hydrolysing agent.

In one embodiment, the hydrolyzing agent is an alkali.

Thus, the method typically comprises the use of an alkoxysilane, particularly tetraalkoxysilanes (abbreviated herein to TAOS). The TAOS's are particularly selected from TEOS (tetraethoxysilane) or TMOS (tetramethoxysilane).

The method typically involves the use of a hydrocarbyl-substituted, particularly an aryl-substituted silane ether, e.g. a silane ether in which one or two of the ether forming groups (RO-) are each replaced by an aryl-containing group, e.g. a phenyl group.

Thus, exemplary substituted silane ethers are of the formula (R'O)ₘ(R²)ₙSi, where R' is an organic residue, R² is an aryl-containing group and m is 2 or 3 whilst n is 1 or 2, provided that (n+m) = 4.

The present disclosure also provides a method for preparing hydrophobic silica particles, the method comprising reacting together in a single step a mixture of TEOS (tetraethoxysilane) and PTEOS (phenyltriethoxysilane) monomers with a hydrolysing agent.

The present disclosure also provides a a method for preparing hydrophobic silica particles, the method comprising reacting together in a single step a mixture of TEOS and PTEOS monomers with an alkali.

The present disclosure also provides a a method for preparing hydrophobic silica particles, the method comprising reacting together in a single step a mixture of (i) TEOS (tetraethoxysilane) or TMOS (tetramethoxysilane) monomers, or a combination thereof and (ii) PTEOS (phenyltriethoxysilane) monomers with a hydrolysing agent e.g. an alkali.

The hydrolysing agent e.g. an alkali act as a catalyst for the reaction between the silane ether monomers, for example, TEOS, and the organically substituted silane ether monomers, for example PTEOS.

The present disclosure also provides a method which comprises incorporating functional agents for imparting post-manufacture functions. The functional agents may aid in the detection, imaging and handling of the particles. In particular, dye molecules, other visualising agents and/or magnetic particles are incorporated into the silica particles. The addition of dye molecules and/or other visualising agents aids in the visualization of fingerprints. The addition of magnetic (or magnetisable) particles may add in the handling of the particles, as described in more detail later.

The present disclosure also provides hydrophobic silica microparticles obtainable by the methods of the present invention. Also provided by the present disclosure are hydrophobic silica nanoparticles obtainable by (having the characteristics of a product obtained by) by the methods of the invention. Preferably, the hydrophobic silica microparticles are coalesced silica nanoparticles.

The present disclosure also provides a a method is provided for producing hydrophobic silica microparticles from silica nanoparticles, wherein the method comprises providing conditions suitable for the nanoparticles to aggregate to form silica microparticles. Typically, the method comprises drying of the nanoparticles to encourage coalescing.

Included in the present invention is the use of hydrophobic silica particles for detecting fingerprints, whether direct or lifted from a surface.

In a further aspect of the present invention, there is provided a method of detecting a fingerprint, either on a surface or lifted from a surface, which comprises contacting an agent comprising hydrophobic silica particles with a fingerprint. The method may optionally comprise imaging the particles when applied to, or contained as part of, a fingerprint.

### Detailed Description of the Invention

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

### Method A

The following method, "Method A", does not form part of the claimed invention. A method for preparing hydrophobic silica particles is provided which comprises reacting together in a single step a mixture of (1) silane ether monomers, for example, a alkoxysilane and (2) organically substituted silane ether monomers, for example a phenyl modified silicate, with a hydrolysing agent e.g. an alkali.

Thus, the method typically comprises the use of alkoxysilane monomers. The method may comprise the use of tetraalkoxysilanes (abbreviated herein to TAOS). The TAOS's are particularly selected from TEOS (tetraethoxysilane) or TMOS (tetramethoxysilane).

The method may comprise reacting together in a single step a mixture of TEOS (tetraethoxysilane) and PTEOS (phenyltriethoxysilane) monomers with a hydrolysing agent. Alternatively, TMOS can be used in place of TEOS in the method.

The method may comprise reacting TMOS monomers and PTEOS monomers.

The mixture may further comprise a water miscible solvent, for example, ethanol, and water.

The method may be carried out at ambient temperature. The duration of the reaction is not critical. The reaction between the TAOS monomers and PTEOS monomers may be performed overnight or for an equivalent time period, that is to say for between about 12 and about 18 hours. The length of the reaction has an effect on the size of silica particles produced. It is believed that the earlier a reaction is stopped, the smaller are the particles which are formed. Therefore, the reaction may be performed over a period of less than 12 hours e.g. between 6 and 12 hours. Alternatively, the reaction may be performed for longer than 18 hours. If desired, the temperature may be elevated (or reduced) and the duration of the reaction reduced (or increased).

The hydrolysing agent, typically an alkali, acts as a catalyst within the reaction. Preferably this catalyst is a hydroxide, for example ammonium hydroxide. The catalyst may be an acid. Examples of acids are mineral acids, e.g. hydrochloric acid. The reaction may comprise an acid induced hydrolysis.

The silane ether monomer, for example a TAOS, and the organically substituted silane ether monomer, e.g. PTEOS monomers may be used, for example, in ratios (PTEOS:TAOS) of from 2:1 to 1:2 e.g. 4:3 to 3:4 and in particular 1.2:1, to 1:1.2. In one class of methods, the ratio is at least about 1:1, e.g. up to 1:5, for example 1:2. In one class of methods, the PTEOS:TAOS ratio is preferably 1:1 v/v. It will be understood that, where one or both of the TAOS and PTEOS are replaced by alternative reagents, the same ratios may be used.

Also disclosed herein, although not claimed is a method for isolating hydrophobic silica particles from a reaction medium formed from the reaction of Method A.

Particles produced by the above method tend to be predominantly nanoparticles, that is to say, of an average diameter of approximately 200nm to about 900nm, typically about 300nm to 800nm and particularly 400nm to 500nm.

These nanoparticles can be subsequently processed to form microparticles, which can be considered coalesced nanoparticles. The microparticles may be produced using Method B:

### Method B

Hydrophobic silica microparticles may also be obtained using a method comprising the steps of;
i) centrifuging a suspension of particles ;
ii) transferring the suspension of hydrophobic silica particles into an aqueous phase;
iii) extracting the suspension from the aqueous phase into an organic phase;
iv) evaporating the organic phase; and
v) crushing and sieving the product obtained in (iv).

Typically, the suspension is a reaction product of a method corresponding to Method A disclosed herein. The hydrophobic silica microparticles may be formed from silica nanoparticles which have been produced from methods other than Method A as disclosed herein.

The organic phase preferably comprises an organic solvent which is non-polar or has low polarity. The organic phase can be e.g. dichloromethane. In alternative embodiments, other organic solvents which can be used as an organic phase include for example alkanes, e.g. hexane, toluene, ethyl acetate, chloroform and diethyl ether.

### Method C

Hydrophobic silica microparticles may also be obtained from a reaction product produced from Method A by a method comprising:
(a) centrifuging the reaction product; and
(b) washing the reaction product in a fluid.

The method can comprise repeating steps (a) and (b) a plurality of times. Preferably, the fluid is an aqueous:solvent mixture and is typically a water:organic solvent mixture. Typically, the organic solvent is ethanol. Preferably the initial fluid comprises a mixture of water and organic solvent at a ratio of from about 60 (water):40 (solvent) to about a 40:60 v/v mixture. The solvent can be, for example, dimethylformamide, n-propanol or iso-propanol.

Typically, the proportion of solvent in the mixture is increased between the initial washing (i.e. suspension) (b) and the final washing (suspension). To obtain microparticles which are coalesced nanoparticles, the final suspension is dried. Preferably, the microparticles are then sieved. Once sieved, the microparticles are ready for application as a fingerprint developing agent.

The particles produced by carrying out Method B and/or Method C are microparticles, that is to say they have an average diameter in the order of micrometers, preferably from about 30 to about 90µm. The microparticles may have an average diameter of from about 45 to about 65µm or from about 65 to about 90µm. The microparticles produced using Method B and/ or Method C are considered to be coalesced nanoparticles.

Thus, disclosed herein is a method for making silica microparticles, e.g. having a diameter of at least 10µm. The method typically comprises:
(i) a first step for making nanoparticles by a technique for controlling particle size, thereby enabling the formation of particles having an average particle size of 500nm +/- 100nm;
(ii) a second step for coalescing the nanoparticles into microparticles of a size suitable for removing by air filters (e.g. face masks) as in the case of microparticles having a diameter of at least 5µm, typically at least 20 µm and preferably at least about 25 to 30 µm.

Typically, step (ii) comprises drying the nanoparticles to encourage coalescing.

### Method D

Hydrophobic silica nanoparticles may alternatively be isolated from a reaction product produced from carrying out Method A. The hydrophobic silica nanoparticles are isolated using a method which comprises centrifuging the reaction product and suspending it in an aqueous:solvent mixture. In one embodiment, Method D is similar to Method C, except Method D does not involve drying the nanoparticles.

The aqueous:solvent mixture may be a first aqueous:solvent mixture. The first aqueous: solvent mixture is preferably a 50:50 mixture.

The method further may comprise removing the reaction product from the first aqueous: solvent mixture, centrifuging it and suspending it in a second aqueous:solvent mixture. Preferably, the second aqueous: solvent mixture has a similar proportion of solvent and aqueous component as the first mixture.

The aqueous solution which forms part of the aqueous:solvent mixture in Method C or Method D may be water.

The solvent which makes up the solvent portion of the aqueous:solvent mixture is, for example, a water-miscible solvent. The solvent may be ethanol. The solvent can be, for example, dimethylformamide, n-propanol or iso-propanol.

The step of suspending the reaction product in an aqueous:solvent mixture and centrifuging it is repeated a plurality of times. Preferably, the composition of the aqueous:solvent mixture is altered to increase the proportion of solvent in the aqueous:solvent mixture over the course of repeated suspensions. Preferably, the method comprises, in the final step, suspending the reaction product in an aqueous: solvent "mixture" which is 0% aqueous:100% solvent. The total number of suspensions is typically from 3 to 10, e.g. 4, 5, 6, 7, 8 or 9. Typically after each suspension except the final suspension the suspensions are centrifuged. The nanoparticles can be stored in the final ethanolic suspension. It will be appreciated that centrifugation is one exemplary method of isolating the nanoparticles from the aqueous:solvent mixture and other separation techniques are not excluded.

The method thus provides a suspension of hydrophobic silica nanoparticles in a solvent. The suspension may comprise trace amounts of the aqueous solution which forms part of the aqueous:solvent mixture.

The hydrophobic silica nanoparticles isolated using Method D are substantially spherical. The nanoparticles isolated preferably, although not essentially, have an average diameter from about 400 to about 500nm. It is envisaged that nanoparticles of a greater or lesser diameter e.g. from about 200nm to about 900nm can be produced by the method. However, it is envisaged that the above method may produce nanoparticles which have an average diameter which is from about 200nm to about 900nm e.g. 200, 250, 300, 350, 400, 450, 500, 550, 600, 650, 700, 750, 800, 850 or 900nm and any combination of sizes to form end-points of ranges. Each of the diameter sizes listed in the previous sentence may be combined with any other of the listed sizes to form end points of a size range of a particular embodiment, thus these are included embodiments in which the nanoparticles have an average diameter of from 200 nm to about 300 n m, from 400 nm to 850 nm, or from 300 nm to 700 nm.

The term "average diameter" can be taken to mean a "mean diameter" of particles typically formed from the methods of the invention. The term "mean" is a statistical term that is essentially the sum of all the diameters measured divided by the number of particles used in such measurements. The diameters of nanoparticles has been estimated from SEM pictures and the scale used in the picture, and for microparticles a combination of the sieve size, the results from particle size distribution measurements and from SEM pictures. One way a mean diameter can be determined is by using an apparatus under the Trade Mark Malvern Mastersizer (Malvern Instruments Ltd.).

Hydrophobic silica nanoparticles isolated using Method D are typically in the form of a suspension in a water-miscible solvent.

### Particles

Also disclosed herein, although not claimed, are hydrophobic silica microparticles obtainable (having the characteristics of microparticles obtained using) using Method B, Method C, Method A and B combined or Method A and C combined. The hydrophobic silica microparticles may comprise trace impurities such as, for example, trace amounts of the hydrolyzing agent e.g. alkali, used in the method. Included therefore are silica particles containing minor amount of component species of the alkali or hydrolysing agent, e.g. ammonium, sodium or potassium. The microparticles may be considered to be aggregates of smaller silica nanoparticles.

The microparticles produced using Method B, Method C, Method A and B combined or Method A and C combined can be used as a dusting agent for fingerprints and the like. In this embodiment, the microparticles are of sufficient size to be efficiently captured using face masks and hence not inhaled. Thus, in one embodiment, the silica microparticles have an average diameter of at least 10 µm, typically at least 20 µm. Typically, the microparticles have an average diameter of from about 30-90µm. In some embodiments, the microparticles have an average diameter of between about 45-65µm or from about 65 to 90µm.

Also enclosed herein is a dusting agent for use in fingerprint detection and/or analysis comprising hydrophobic silica microparticles. Alternatively, the microparticles may be suspended in an ethanolic aqueous mixture.

However, the preferred form of an agent comprising microparticles is a dry particulate form, since the microparticles tend to sink in a suspension.

Also disclosed herein, is a suspension of hydrophobic silica nanoparticles in a solvent obtainable by Method D. The solvent may be ethanol. The solvent may be another water-miscible solvent eg. one exemplified herein. The suspension may comprise a trace amount of the aqueous component of the aqueous:solvent mixture used in the method.

The physical nature and dimensions of the particles may be determined using SEM and TEM scans. The particles are in the form of amorphous silica which is commonly used as an anti-caking agent in a variety of food products, and as an anti-caking agent and as an excipient in pharmaceuticals for various drug and vitamin preparations [12].

The nanoparticles formed from the methods described herein can be applied in a suitable liquid medium to latent fingerprints or to a surface to determine whether a fingerprint is present. Typically, the suspension of nanoparticles in solvent produced by the method for isolating silica nanoparticles is diluted with an aqueous component before use as a fingerprint detecting agent. The article in which a print is deposited may be immersed in the liquid medium (i.e. suspension of nanoparticles) and then removed. The length of immersion is not critical and may vary from about 15 minutes to about 12 hours or longer.

Thus, in a preferred embodiment, the nanoparticles are applied to a fingerprint or surface in a suitable liquid medium. Typically, the liquid medium is an aqueous:solvent mixture. Also provided is a suspension comprising hydrophobic silica nanoparticles, an aqueous component and a water-miscible solvent. In one embodiment, the aqueous component is water. The solvent may be for example a water miscible solvent i.e. 100% miscible in all proportions in water. In one embodiment, the solvent is ethanol. The water: solvent ration ranges from about 99.9:0.1 (water:solvent) to about 96:4 (water: solvent). The level of solvent preferably is not greater than about 4%, since a higher level of solvent may result in the fingerprints becoming dissolved or their definition reduced. It is preferable to include at least a trace amount of solvent to ensure that the nanoparticles remain as discrete particles and do not coalesce to form aggregates.

It will be understood that the term "fingerprint" includes reference to a partial print and/or to prints of other body parts, and that, for example, the silica particles may be applied to a portion of a fingerprint. Typically, a fingerprint is lifted from its underlying surface prior to analysis, and the term "fingerprint" accordingly includes lifted fingerprints. In embodiments, the fingerprint is lifted prior to application of the particles.

In this embodiment, the hydrophobic silica nanoparticles should not form aerosols during the application process and therefore should be safe to apply. It should also be noted that the nanoparticles are too large to cross biological membranes such as skin and lung tissue. Hence both forms i.e. microparticles and nanoparticles, should be safe agents when used in the contexts described above.

The methods described above produce a range of stable silica micro- and nanoparticles. However in order to be able to visualise these particles it is advantageous to incorporate a variety of dyes within them.

Thus, the methods may further comprise incorporating a dye or other imaging agent into the silica particle. To achieve incorporation of small molecules such as dyes (for example coloured or fluorescent), functional groups are incorporated within the particle, forming strong binding interactions with the dye molecules. These functional groups are either hydrophobic or hydrophilic, or combinations of both.

The functional groups are present in one or more of the silane ether monomers and it is presumed that, when the dye molecules are added, interactions are formed between the dye molecules and appropriate functional groups provided by the monomers to maximise the interactions between the two components of the mixture.

Without being bound by scientific theory, it is believed that addition of the catalyst, for example ammonium hydroxide, results in polymerisation and the dye-backbone interactions are frozen resulting in pockets of folded backbone in which are located the dye molecules. The dye molecules cannot be easily extracted using aqueous solutions from the resulting particles and this may be due to the compact folding of the backbone around the dye molecules which results in pores that are smaller than the dye molecules themselves, or in substructures in which the binding interactions. One example of such binding interactions is hydrophobic interactions between the planar aromatic groups within the polymer of PTEOS derived particles and the dye molecule. Other examples are ionic interactions between positively charged groups on the dye molecules and the adjacent negatively charged groups; for example, Si-O groups on the polymer backbone are sufficiently large to retain the dyes within the matrix even in the presence of large diameter pores.

As described earlier, the ratio of silane ether monomers, for example, TEOS and organically substituted silane ether monomers, for example PTEOS is preferably about 1:1 v/v. It is at this ratio that the optimum incorporation and therefore retention of a dye molecule within the silica particle is demonstrated. However, it will be understood by the skilled person that other ratios can be used which will result in the incorporation and retention of the dye molecule, and therefore enable the detection of the particle.

In an embodiment, the dye to be incorporated into the particle can be for example a coloured or a fluorescent dye. Examples of dyes included in the scope of the invention are , although not limited to, fluorescein derivatives for example Oregon Green, Tokyo Green, SNAFL, and carboxynapthofluorescein, rhodamine (e.g rhodamine B and rhodamine 6G) and analogues thereof, thiazole orange, oxazine perchlorate, methylene blue, basic yellow 40, basic red 28, and crystal violet,and analogs thereof,. Without being bound by scientific theory, it is considered that dyes which are positively charged, for example, rhodamine, are better incorporated when PTEOS is used in the method than dyes which comprise anionic or cationic group such as carboxylic groups. Examples of other dyes which could be used in the invention include those which possess a planar aromatic substructure and positively charged functional groups (e.g. ethidium bromide and other DNA intercalating agents).

Preferably the dyes are incorporated from aqueous solutions during the single-step reaction step described above (Method A), that is to say, the dye is included in the reaction mixture of TAOS (e.g. TMOS and/or TEOS) and PTEOS monomers.

It may be advantageous for the particles to be magnetic or paramagnetic. For example, magnetisable microparticles can easily be dusted over fingerprints, using a magnetic wand or other appropriate tool. In a preferred embodiment of the invention therefore, magnetic or para-magnetic subparticles are incorporated into the monomer mixture in Method A. In an embodiment of the invention, the particles of the invention are magnetisable e.g. magnetic or paramagnetic.

In one embodiment, the methods for preparing hydrophobic silica particles further comprise including magnetic or paramagnetic particles in a reaction mixture of silane ether monomers, for example TAOS (e.g. TEOS) monomers and organically modified silane ether monomers, for example, PTEOS monomers. Thus, preferably the magnetic or paramagnetic particles are incorporated during the single-step reaction step described above (Method A). The magnetic and/or paramagnetic particles may be any magnetic or paramagnetic component, for example, metals, metal nitrides, metal oxides and carbon. Examples of magnetic metals include iron, whilst examples of a metal oxide include magnetite. Carbon may be in the form of, for example, carbon black, fullerene or carbon nanotubes (derivatized or non-derivatized carbon nanotubes). The carbon nanotubes may be multi-walled carbon nanotubes and/or single walled carbon nanotubes.

Hydrophobic silica particles containing both a magnetisable material and also a separate imaging material (particularly a dye e.g. a fluorescent or coloured dye) are novel and form part of the invention.

In one embodiment, the magnetisable material may be magnetic or paramagnetic particles comprising, for example, magnetite, which is included in the reaction mixture of silane ether monomers, for example TAOS (e.g. TEOS) monomers and organically modified silane ether monomers, for example, PTEOS monomers. "Pale brown" particles are formed when haematite is included in the mixture. Black particles are formed when magnetite is included in the mixture. Both types of incorporated particles are found to be highly magnetisable.

In some embodiments, Method A comprises included, for example, titanium dioxide or carbon black in the reaction mixture. Other examples of "white" or "grey" particles are formed when either titanium dioxide or Carbon Black particles respectively are included in the reaction mixture of (1) silane ether monomers, for example TAOS (e.g. TEOS) monomers and (2) organically modified silane ether monomers, for example, PTEOS monomers, mixture during synthesis. The relatively large size of these particles results in their entrapment within the polymer matrix whilst producing the required hydrophobic silica particles. The colour of the "grey" particles is dependent on the amount of carbon black or titanium dioxide included in the TAOS (e.g. TEOS)/ PTEOS mixture during synthesis. A higher level of carbon black or titanium dioxide results in a darker particle.

As an alternative, or in addition, to the particles comprising a magnetisable element, the hydrophobic silica particles can be mixed with a magnetic substance, for example, iron filings to form a magnetisable agent which can be applied to finger prints.

The prior art methods of fingerprint detection include applying a magnetic substance, include for example, using iron particles which are coated with a hydrophobic substance such as stearic acid. This form of fingerprint detection often has the disadvantage that the iron particles can be rubbed off onto a fingerprint, thus causing depletion of the iron and thus reducing the amount available for future administration. One advantage of incorporating magnetic particles into the silica particles during synthesis is that the level of magnetic substance to be applied remains constant, since the amount of magnetic particle per silica particle tends not to alter significantly.

In a disclosed method for preparing hydrophobic silica particles, using a hydrolyzing agent e.g. an alkali comprises mixing magnetic micro- or nanoparticles are mixed with iron filings to generate a range of magnetisable particles.

Also disclosed herein is a hydrophobic silica particle obtainable by the methods disclosed herein or which has the characteristics of a particle made by the methods disclosed herein. The particle can be a hydrophobic silica microparticle.

Also provided is a hydrophobic silica nanoparticle having a diameter of between about 200 to about 900 nm, typically about 300 to 600 nm and more typically about 400 nm to about 500 nm.

The hydrophobic silica particle (microparticle and/or nanoparticle) may comprise a dye. Examples of such dyes are described elsewhere in the specification. The dye may be coloured or fluorescent and can provide visualisation means which enable the particles to be seen.

The silica particle may further comprise a magnetisable element, for example, a magnetisable particle. The particle may comprise magnetite and/or haematite.

The silica particle may comprise a dye and a magnetisable element.

The silica particle may comprise a metal, metal oxide, metal nitride and/or a carbon molecule e.g. carbon black and/or titanium dioxide, either alone or in combination with a dye and/or magnetisable element, for example magnetite.

The hydrophobic silica particles can be used in the process of fingerprint detection and identification. Thus, disclosed herein is use of a hydrophobic silica particle as described herein, for example, a silica particle obtainable by the methods described herein, in the detection and/or identification of a fingerprint. Hydrophobic silica particles which have the characteristics of a particle may be obtained by one or more of the methods described herein.

Such a use may comprise imaging a fingerprint.

Also provided is the use of the hydrophobic silica micro- and nanoparticles for fingerprinting. The silica particles may be used as agents for dusting and/or developing a fingerprint.

Thus, there is provided an agent for dusting a fingerprint comprising hydrophobic silica particles. The particle may be a microparticle. The agent may include hydrophobic silica nanoparticles.

The agent for dusting may comprise hydrophobic silica particles which comprise a dye molecule. The dusting agent may comprise hydrophobic silica particles which comprise a magnetisable element. Thus, the silica particles may be magnetic or paramagnetic. The dusting agent may comprise hydrophobic silica particles which comprise both a magnetisable element and a dye molecule. Alternatively, the agent may comprise a mixture of hydrophobic silica particles and a magnetisable material, for example, iron filings.

A latent fingerprint, deposited by the fingertip pattern, is a complex mixture of natural secretions and contaminations from the environment. The latent prints can be fresh or aged finger prints. The residues within aged prints mainly consist of hydrophobic endogenous chemicals secreted by the donor. Thus, the dusting agent of the present invention may be used to detect and/or identify both fresh and aged fingerprints.

In a further aspect of the invention, there are provided hydrophobic silica microparticles comprising aggregates of nanoparticles. The microparticles of the present invention can be used as dusting agents, e.g. in the form of a powder, for latent prints on a variety of porous and semi-porous surfaces. The microparticles can be applied using standard commercial brushes or via a commercial magnetic wand for magnetic particles. If the microparticles incorporate magnetic subparticles and/or have been mixed with iron filings (to generate a range of magnetisable particles) then these particles can be applied to the prints using a magnetic wand. The microparticles may additionally comprise a dye and/ or coloured particle.

In an embodiment, the silica microparticles or nanoparticles can be applied directly to the surface as a suspension. Thus, in one aspect of the present invention, there is provided a suspension for detecting fingerprints comprising silica particles of the present invention. In one embodiment, the suspension comprises water and ethanol. This is particularly advantageous as it allows objects e,g weapons to be submerged in the suspension, thus increasing the likelihood of detecting a latent print on any surface of the object.

Also disclosed herein is the use of a hydrophobic silica particle as an agent for developing a deposit eg. latent fingerprint on a surface. The use may further comprise imaging the fingerprint.

Also disclosed herein is a method of detecting and/or identifying fingerprints comprising contacting a fingerprint with an agent comprising the silica particles of the present invention.

Typically, the method further comprises visualizing and/or imaging the fingerprint.

The agent may be a dusting agent. The agent may be a suspension as described herein. When the agent is a suspension, the method may further comprise a step of drying the fingerprint prior to visualizing the fingerprint.

The step of visualizing the fingerprints may be carried out using various methods known in the art. For example, optical methods may be used, for example, a UV search light, optical scanner including a flat-bed optical scanner, a fluorescent scanner and a UV visible scanner.

The invention will now be described without limitation with reference to the accompanying examples and figures.

### Brief description of the Figures

Figure 1: SEM for carbon black embedded nanoparticles
Figure 2: SEM for carbon black embedded microparticles
Figure 3: Size distribution for hydrophobic micro-particles with embedded carbon black obtained through a 38-63µm mesh.
Figure 4a: Electron diffraction patterns for amorphous silica nanoparticles.
Figure 4b: Electron diffraction patterns for α-quartz
Figure 5: Structures of coloured and fluorescent dyes.
Figure 6: Prints developed with rhodamine 6G-incoporated particles. Applied as a nanoparticle suspension (upper) and as a dusting powder (lower); left; white light illumination: right:UV illumination.
Figure 7: Detail of 40-day old print dusted with crystal violet-incorporated micro-particles (left) and a print from the same donor developed with a suspension of crystal violet-incorporated nanoparticles.
Figure 8: Examples of dusted aged prints on glass, with incorporated sub-particles (upper) or dyes (lower)

### Materials and Methods

### Materials

Ethanol (99.7%) was purchased from Hayman Ltd., UK. Tetraethoxysilane (98%+), crystal violet, thiazole orange, oxazine 1 perchlorate and titanium dioxide, were purchased from Aldrich, Dorset, UK. Phenyltriethoxysilane was supplied by Fluorochem, Derbyshire UK. Bromothymol blue and methylene blue were purchased from BDH Chemicals Ltd, now VWR International Ltd., Leicestershire, UK. Ammonium hydroxide solution (28%), sodium fluorescein, Rhodamine B and Rhodamine 6G was purchased from Sigma-Aldrich, Dorset, UK. The carbon black solution was a kind donation from Cabot Corp., Cheshire, UK. The nanoparticulate magnetite was synthesised in-house according to previously published methods [10-11].

### Preparation of TEOS:PTEOS-derived particles.

The basic method for the preparation of blank micro- or nanoparticles involves mixing; 30 ml ethanol, 5 ml dH₂O, 2.5 ml each of tetraethoxysilane (TEOS) and 2.5 ml phenyltriethoxysilane (PTEOS) in a centrifuge tube. To this mixture add 2 ml ammonium hydroxide solution and rotate the solution overnight. After this time, centrifuge the suspension (3 minutes at 3,000 rpm).

### Method 1

This method yields microparticles. The method involves the centrifugation and liquid/liquid phase extraction into dichloromethane from water, followed by evaporation of the organic phase to dryness, yielding a glass-like sheet of coalesced particles. This is crushed using a mortar and pestle and the resulting particles sieved through brass test sieves with bronze mesh (Endecot Ltd., London UK) by hand.

The particle size fractions used in this study are 38-45, 45-63 and 63-90 µm and these were each used as dusting agents. A Malvern Mastersizer (Malvern Instruments Ltd., Malvern, UK) can be used to verify the particle size distributions. Due to the hydrophobicity of the particles, ethanol is used as the solvent. A Hitachi Scanning Electron Microscope (SEM) is used to visualise the particles and a Hitachi Transmission Electron Microscope (TEM) is used for electron diffraction patterns.

### Method 2

This method yields nanoparticles. The product is isolated following a series of centrifugation and washing steps using 10:90 v/v ethanol/water and then retained as a suspension in 97:3 v/v water/ethanol. These were also subjected to particle size distribution analysis and SEM and TEM.

### Method 3

This method yields nanoparticles. The product is isolated following two washes with a 50:50 mixture of water:ethanol, followed by a wash using 25:75 mixture of water:ethanol and a final wash using 0:100 mixture of water:ethanol. The reaction product was then resuspended in as little ethanol as possible to transfer to a drying dish. The suspension was left at room temperature for several days before being kept at a temperature of 37°C for several days.

For the different dye doped particles, the relevant dye (25 mg) is dissolved into the ethanol prior to the addition of the other silanisation reagents. The titanium dioxide embedded particles are prepared by adding 25 mg of titanium dioxide to the centrifuge tube, prior to the addition of the silanisation reagents. For carbon black particles, 5 ml of a 1:100 fold dilution of the carbon black suspension in water is added to the precursor solution. For TEOS:PTEOS coated magnetic particles, particulate magnetite is prepared according to published methods and 5 ml of the suspension in water is included in the precursor solution.

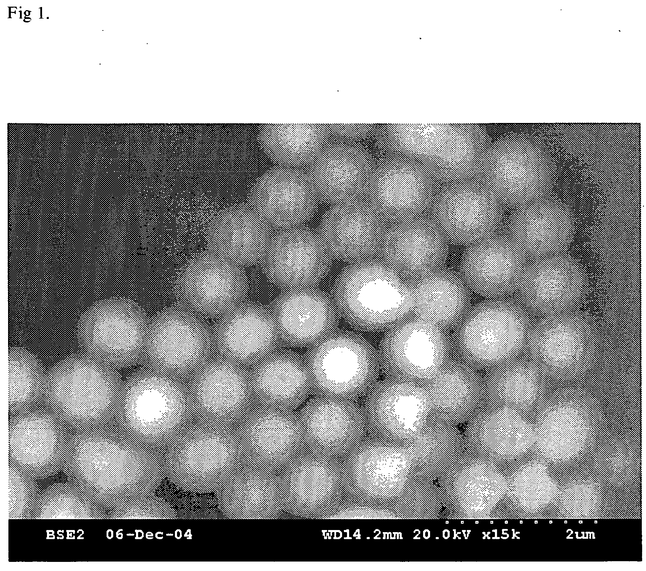

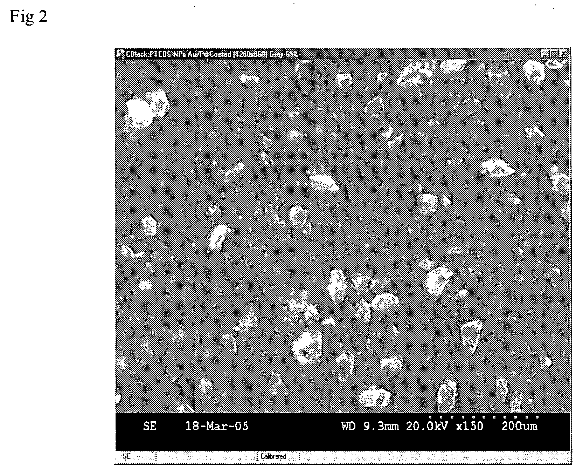

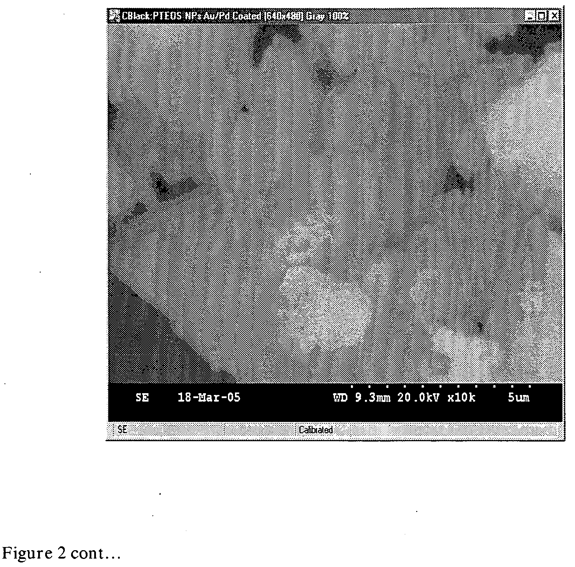

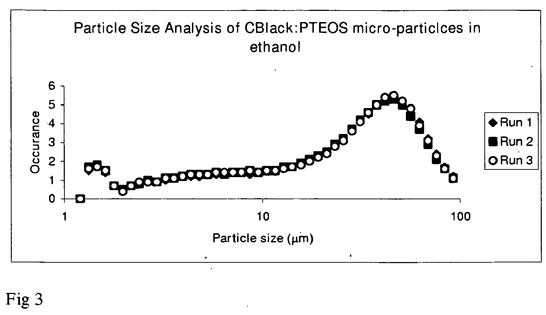

Using the two methods described above a range of microparticles and nanoparticles were produced. SEMs are shown for carbon black-embedded nanoparticles (Figure 1) and carbon black-embedded microparticles (Figure 2). The nanoparticles are composed of discrete spherical particles with an average diameter of 400-500 nm, whereas the micro-particles consist of aggregates of nanoparticles with an average diameter of about 27 µm for the 38-63 µm-sieved fraction (Figure 3).

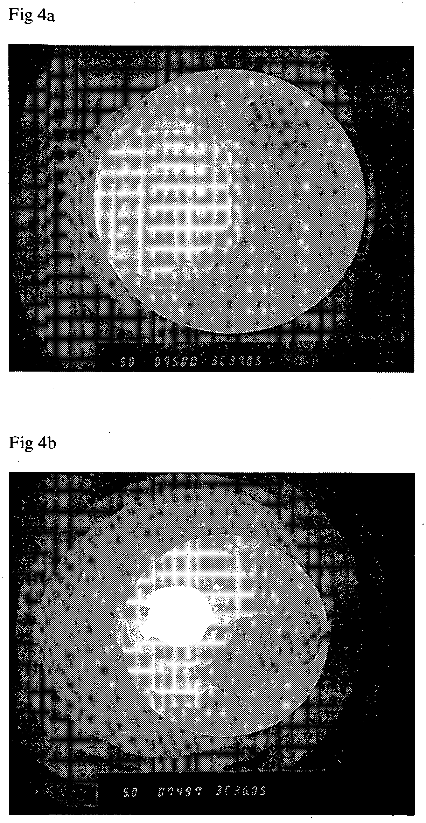

The TEM data (Figure 4a) show no regular diffraction pattern for the silica nanoparticles demonstrating that that the particles consist of amorphous, non-crystalline silica. This is in contrast to the diffraction pattern observed with crystalline silica (Figure 4b).

A variety of fluorescent and coloured hydrophobic particles were prepared. In addition a variety of white, grey and magnetic sub-particles were also incorporated within the hydrophobic silica micro-particles. The relatively large size of these particles resulted in their entrapment within the polymer matrix whilst producing the required hydrophobic silica particles. White particles were formed when titanium dioxide was included in the TEOS/PTEOS mixture, grey particles when carbon black was included, and pale brown when haematite was included or black when magnetite was included. The magnetite incorporated and haematite incorporated particles were found to be highly magnetisable.

### Example 1

**The effect of TEOS:PTEOS ratios on incorporation of ethidium bromide and dyes**

This was performed using the method as detailed above, with ratios of TEOS:PTEOS varying from 1:0, 0.9:0.1, 0.8:0.2 and 0.7:0.3. Ethidium bromide, crystal violet, bromothymol blue and analogs of rhodamine were used to test the mechanism for effective dye incorporation.

### (i) Ethidium bromide (EtBr)

Ethidium bromide (Figure 4) is used extensively as a fluorescent marker for DNA with which it forms a highly fluorescent complex due to its intercalation between base pairs of the DNA α-helix. Such intercalation results from a combination of strong hydrophobic bonds between the planar base pairs within the interior of the DNA chains and the planar aromatic rings of the dye, and electrostatic bonds between the negatively charged phosphate groups of DNA and the positively charged group of the dye. The planar aromatic phenyl groups of PTEOS form hydrophobic interactions with the planar rings of EtBr and the negatively charged Si-O groups present in PTEOS and TEOS form electrostatic bonds with the positively charged nitrogen of ETBr thus simulating the intercalation seen with DNA, and thus producing a highly fluorescent and stable complex on polymerisation when nanoparticles are formed. Nanoparticles formed with TEOS and EtBr were of low fluorescence since the dye was washed from the particles following their formation. In contrast when PTEOS was added, highly fluorescent particles were formed which retained the dye on washing. It was also noted that the intensity of the dye's fluorescence increased as the proportion of PTEOS in the initial monomer mixture increased. A ratio of 1: 1 v/v was used thereafter in all incorporation experiments. However it was found that this dye is prone to photo-bleaching under exposure to high intensity UV illumination and hence was not a suitable reporter system for fingerprint development work.

### (ii) Crystal violet (CV) and bromothymol blue (BTB),

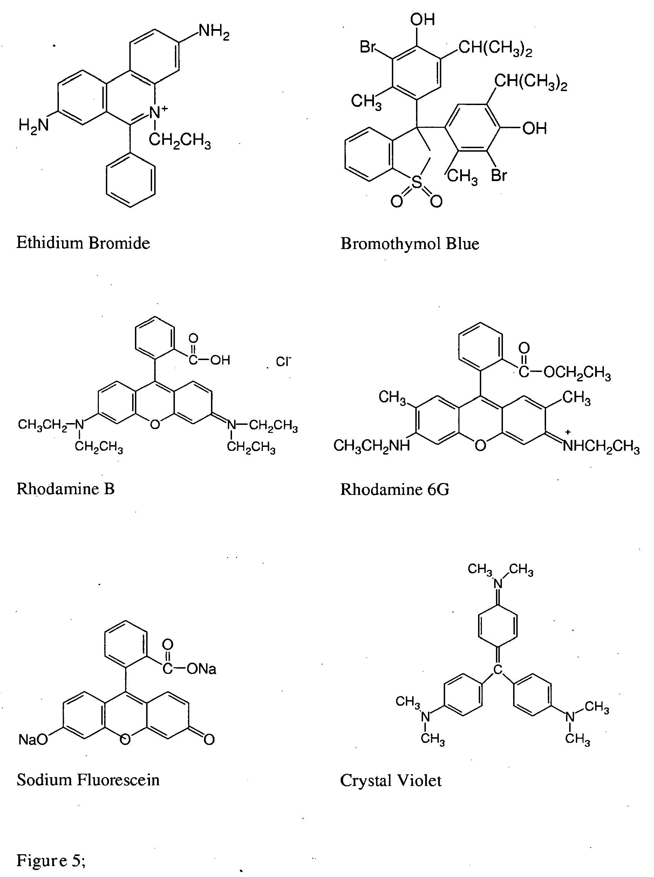

Crystal violet (CV) and bromothymol blue (BTB), were independently included in a mixture 1:1 mixture of PTEOS and TEOS monomers. Both dye molecules are similar to EtBr in that they contain planar phenyl groups but CV contains a quaternary ammonium group and secondary amino groups similar to those present in EtBr, whereas BTB is an acidic molecule having phenolic and sulphonic acid groups (Figure 5). CV-incorporated nanoparticles produced particles with a dark purple colour. This may be due to the amino groups in CV producing strong attractive interactions with the silane residues. In comparison the BTB particles were off-white as the BTB dye was not found to be retained within the silica nanoparticles on workup, possibly due to repulsion between the negatively ionised acidic groups of BTB and the negatively charged Si-O groups within the monomers.

### (iii) Analogs of rhodamine

Rhodamine B yielded a dark red powder whilst rhodamine 6B yielded an orange powder (not shown), both of which were intensely fluorescent.

### Example 2

### The effect of APTES on incorporation of dyes into TEOS/PTEOS sol gels

This was performed using the above method for the preparation of TEOS:PTEOS particles. In addition the same dyes were used but APTES was incorporated into the monomer mixture. Here, a ratio of 3:3:6 of PTEOS:APTES:TEOS was used (1.25ml:1.25ml:2.5ml) instead of the usual (2.5ml:2.5ml) PTEOS:TEOS ratio. The dyes used were fluorescein, thiazole orange, oxazine perchlorate, methylene blue, basic yellow 40 and basic red 28. In addition, two types of rhodamine were used, rhodamine B and rhodamine6G. In each case, the dye-doped particles were isolated as ground and sieved micro-particles.

A wide of stable dye-doped particles were formed. In each case, the dyes were incorporated into micro-particles from aqueous solutions, using the PTEOS and TEOS method. For example Rhodamine B yielded a dark red powder whilst rhodamine 6B yielded an orange one. Both were intensely fluorescent. In contrast when APTES was included in the mixture of monomers, the resulting powder was pale pink and of low residual fluorescence. The same phenomenon was observed for sodium fluorescein when addition of APTES again resulted in poor incorporation of the fluorescent dye and producing a barely fluorescent off white powder. These results indicate that the presence of the amino groups in the silica particles destabilises dye-polymer interactions producing particles from which the dyes can be extracted under aqueous work-up conditions.

### Example 3

### The use of dye-doped hydrophobic nanoparticle suspensions as developing agents for latent fingerprints

Both fresh (approx. 20 min prior to dusting) and aged prints (various conditions detailed within) were studied. The fingerprints were deposited by a 21 year old Caucasian female and a 33 year old Caucasian male onto non-porous glass microscope slides (VWR Int,), used supplied. Two methods were used for development. A small volume of suspension (500 µl of a 10% w/v in 97:3 v/v water/ethanol) was applied to the print using a dropping pipette. After 2-3 min the excess suspension was removed by gently washing with excess water. The print was then left to air dry. Alternatively the slide was immersed in the suspension for 2-3 min. The excess developer was removed by gravity and the surface allowed to air dry as before.

The resulting fingerprints were visualised by different optical methods. For the fluorescent observations, a hand held UV search light (λ 415 nm) from CSI Ltd. was used. For further fluorescent imaging and capture, a Tecan LS300 optical scanner (Tecan UK, Theale, UK) was used. The developed fingerprints were also digitally captured using a Nikon Coolpix 5400 camera.

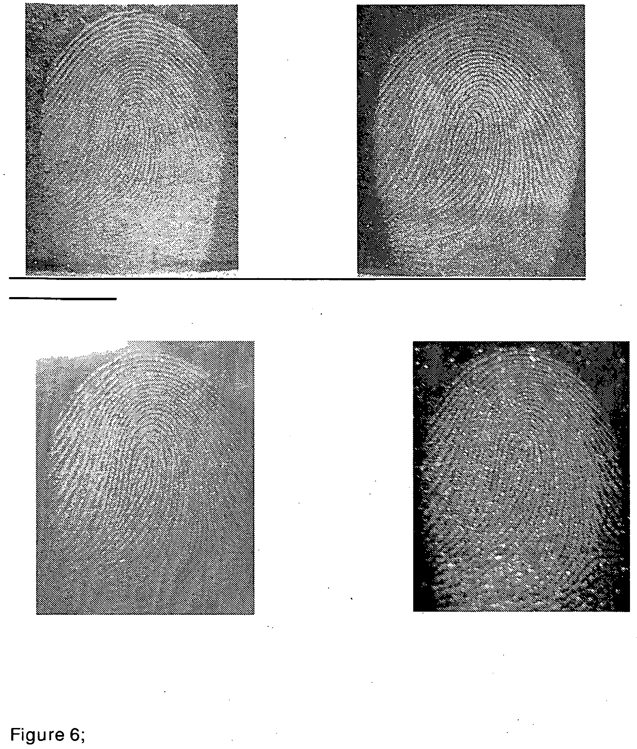

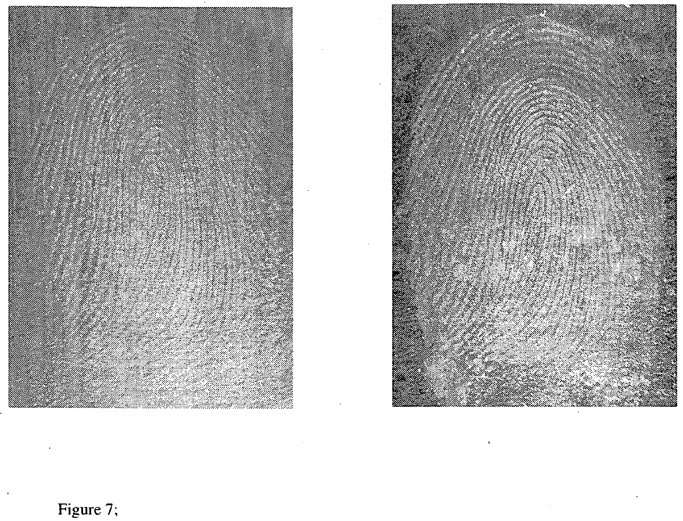

Both fluorescent and coloured nanoparticles were successfully used as developing agents for both fresh and aged prints. Examples of the former are shown in Figure 6 (upper) for particles with incorporated rhodamine 6G, and of the latter in figure 7 (right) for particles with crystal violet-incorporated dye. Excellent definition was seen in both cases under white light illumination and under UV illumination for the fluorescent particles (Figure 6 upper, right).

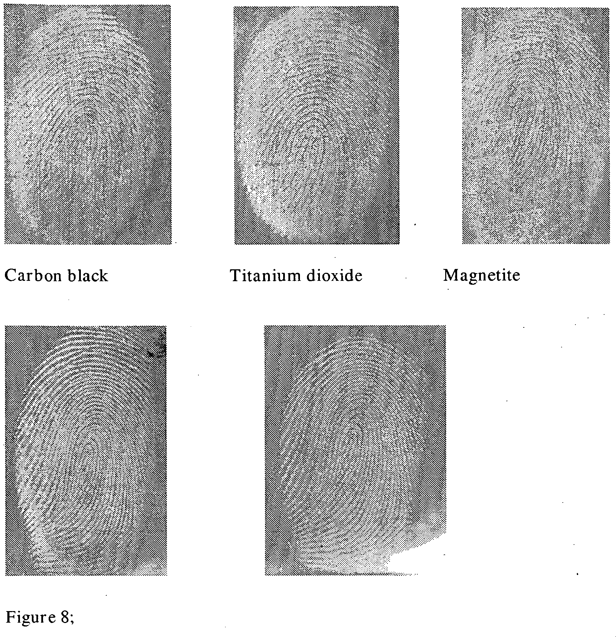

Examples of prints obtained by conventional dusting with microparticles incorporating carbon black, titanium dioxide, magnetite, crystal violet, and methylene blue are shown in Figure 8. Excellent definition was observed with all the particles demonstrating the universality of the approach. However for dusting, particles of diameter 45-63 micrometers gave the best results.

### Example 4

### The use of dye-doped hydrophobic micro-particles as dusting agents for latent fingerprints

The dye-doped particulate powders developed for this study were brushed onto the latent fingerprints using either a squirrel hair brush or a glass fibre (Zephyr) brush. The carbon black and titanium dioxide containing particles were applied using a glass fibre (Zephyr) brush. The in-house developed magnetic powders were applied with commercial plastic magnetic brushes (Crime Scene Investigation Equipment Ltd., Northampton, UK).

The resulting fingerprints were visualised by different optical methods. For the fluorescent observations, a hand held UV search light (A 415 nm) from CSI Ltd. was used. For further fluorescent imaging and capture, a Tecan LS300 optical scanner (Tecan UK, Theale, UK) was used. The developed fingerprints were also digitally captured using a Nikon Coolpix 5400 camera.

A variety of fractions from sieving were used as dusting agents. The one which gave greatest ease of application and produced prints of best definition were those with from sieve size 45-63 µm for fluorescent and coloured particles and 63-90 µm for the sub-particle embedded particles. Examples of prints developed with fluorescent dye (rhodamine 6G) is shown in figure 6 (lower), under white (left) and UV (right) illumination. Figure 7 shows a dusted print from a 40 day old sample on glass produced by applying crystal violet-incorporated dye (left). Generally better definition was observed with the fluorescent and dye-doped particles (Figure 8) but the embedded particles still produced prints with good definition.

The following paragraphs form part of the present disclosure but do not form part of the claimed invention:
1. A method for preparing hydrophobic silica particles, the method comprising reacting together in a single step a mixture of TEOS and PTEOS monomers with a hydrolysing agent.
2. A method for preparing hydrophobic silica particles, the method comprising reacting together in a single step a mixture of TEOS and PTEOS monomers with an alkali.
3. A method according to paragraph 2, wherein the alkali is a hydroxide.
4. A method according to paragraph 3, wherein the hydroxide is ammonium hydroxide.
5. A method according to any of paragraphs 1 to 4, wherein the hydrophobic silica particles are isolated from the reaction medium.
6. A method according to paragraph 5, wherein hydrophobic silica microparticles are isolated using a method comprising the steps of;
   a. centrifuging the reaction product;
   b. extracting the reaction product from an aqueous phase into an organic phase;
   c. evaporation of the organic phase and;
   d. crushing and sieving of the product obtained in (iii).
7. A method according to paragraph 6 wherein the organic phase is dichloromethane.
8. A method according to paragraph 7, wherein the microparticles have an average diameter of between about 30-90µm.
9. A method according to paragraph 8, wherein the microparticles have an average diameter of between about 45-65µm.
10. A method according to paragraph 9, wherein the microparticles have an average diameter of between about 65-90µm.
11. A method according to paragraph 5, wherein hydrophobic silica nanoparticles are isolated using a method comprising the step of suspending the reaction product in an aqueous:solvent solution.
12. A method according to paragraph 11, wherein the solvent is ethanol.
13. A method according to paragraph 10, wherein the nanoparticles have an average diameter of 400-500nm.
14.A method according to any of paragraphs 1 to 13, wherein the ratio of TEOS:PTEOS is 1:1 v/v.
15.A method according to any of paragraphs 1 to 14, wherein a dye molecule is incorporated into the reaction product.
16.A method according to paragraph 15, wherein the dye molecule is bound to a functional group on the TEOS or PTEOS monomer.
17. A method according to paragraph 16, wherein the functional group is hydrophobic.
18. A method according to paragraph 17, wherein the functional group is hydrophilic.
19. A method according to paragraph 16 wherein the functional group on the TEOS monomer is a negatively charged Si-O group.
20. A method according to paragraph 16 wherein the functional group on the PTEOS monomer is a planar aromatic phenyl group.
21. A method according to any of paragraphs 1 to 18, wherein a magnetic of paramagnetic sub-particle is incorporated into the reaction product.
22. A method according to paragraph 19, wherein titanium dioxide, carbon black or magnetite has been added to the mixture of TEOS/PTEOS monomers.
23. The use of hydrophobic silica particles prepared by a method according to any of the preceding paragraph as an agent for developing a latent fingerprint on a surface.
24. The use according to paragraph 23, wherein hydrophobic silica particles are applied to the surface within a dust or a suspension.
25. Use of a hydrophobic silica particle as an agent for developing a latent fingerprint on a surface.
26. The use according to paragraph 25, wherein hydrophobic silica particles are applied to the surface within a dust or a suspension.

### References

1.Champod, C., Lennard, C., Margot, P. & Stoilovic, M. in Fingerprints and Other Ridge Skin Impressions, CRC Press, Boca Raton, 2004 (ISBN 0-415-27175-4)
2. G. S. Sodhi and J. Kaur, Powder Method for Detecting Latent Fingerprints: A Review Forensic Sci. Int., 120 (2001) 172-176
3. E. R. Menzel, S. M Savoy, S. J. Ulvick, K. H. Cheng, R. H. Murdock and M. R. Sudduth, Photoluminescent Semiconductor Nanocrystals for Fingerprint Detection, Journal of Forensic Sciences (1999) 545-551
4. E. R. Menzel, M. Takatsu, R. H. Murdock, K. Bouldin and K. H. Cheng, Photoluminescent CdS/Dendrimer Nanocomposites for Fingerprint Detection, Journal of Forensic Sciences (2000) 770-773
5. E. R. Menzel, Functionalized Europium Oxide Nanoparticles for Fingerprint Detection - A Preliminary Study, J. Forensic Ident.
6. Rowell F. J. and Theaker B.T., Nanoparticle Fingerprint System, UK Patent Application No. 0400235.8
7. New patent for Single step Process, UoS
8. Rao, A.V., Kalesh, R.R. and Pajonk, G.M., J. Materials Science, 38, 4407-4413 (2003)
9. Tapec, R., Zhao, X.J. and Tan, W., Development of Dye-doped silica nanoparticles for bioanalysis and biosensors, Journal of Nanoscience and Nanotechnology, 2, 405-409 (2002).
10. J. J. Harburn, R. R. Ritter, C. D. Spilling, K. M. Miller, Magnetically Responsive Particles and Embolic Materials using Coated Magnetically Responsive Particles, US Patent US60397996, (2004).
11. L. A. Harris, J. D. Goff, A. Y. Carmichael, J. S. Riffle, Magnetite Nanoparticle Dispersions Stabilized by Triblock Copolymers, Chemistry of Materials, 15 (2003) 1367
12. Expert Group on Vitamins and Minerals Secretariat, Review of Silican, August 2002, Food Standards Agency, www.food.gov.uk/multimedia/pdfs/evm0107p.pdf
13. The Royal Society and The Royal Academy of Engineering Report on Nanosciences and nanotechnologies, Chapter 5, Possible adverse health, environmental and safety aspects, July 2004, http://www.nanotec.org.uk

## Claims

1. A method for detecting and/or identifying a fingerprint or other hydrophobic mass or deposit, comprising contacting an area where there is or maybe a fingerprint with an agent comprising hydrophobic silica particles, wherein the method comprises applying the agent as a dusting agent or as a suspension which comprises water and a water-miscible solvent to the area.

2. A method according to claim 1, wherein the silica particles are obtainable by a method comprising reacting together in a single step a mixture of silane ether monomers and organically modified silane ether monomers with a hydrolysing agent.

3. A method according to claim 2, wherein the silica particles are obtainable by a method in which the silane ether monomers are TAOS (tetraalkoxysilane) monomers.

4. A method according to claim 2 or claim 3, wherein the silica particles are obtainable by a method in which the organically modified silane ether monomer comprises an aryl containing group.

5. A method according to any of claims 2 to 4, wherein the silica particles are obtainable by a method which comprises reacting together in a single step a mixture of TEOS (tetraethoxysilane) and PTEOS monomers (phenyltriethoxysilane) with the hydrolysing agent.

6. A method according to any of claims 2 to 5, wherein the silica particles are obtainable by a method in which the hydrolysing agent is an acid or an alkali.

7. A method according to claim 6, wherein the silica particles are obtainable by a method in which the alkali is a hydroxide, and is optionally ammonium hydroxide.

8. A method according to any of claims 2 to 7, wherein the silica particles are obtainable by a method in which dye molecules and/or coloured particles are included in the mixture of the monomers and the hydrolysing agent and are thereby incorporated into the hydrophobic silica particles.

9. A method according to any preceding claim, further comprising visualizing or imaging the fingerprints, wherein the silica particles comprise a visualization or imaging agent and the method comprises visualizing and/or imaging the particles after the surface has been contacted with them.

10. A method according to claim 9, wherein the particles comprise a dye molecule for imaging the fingerprint.

11. A method according to claim 10, wherein the dye is a fluorescent dye.

12. A method according to claim 10, wherein the silica particle comprises a dye molecule and/or coloured particles, which are selected from rhodamine B, rhodamine 6G, carbon black, titanium dioxide, magnetite, crystal violet, and methylene blue.

13. A method according to any preceding claim, wherein the particles comprise a magnetic or paramagnetic particle which enables the agent to be applied to the area using a magnetic applicator.

14. A method according to claim 13, wherein the silica particles are obtainable by a method in which the magnetic and/or paramagnetic particle are included in the mixture of the monomers and the hydrolysing agent and are thereby incorporated into the hydrophobic silica particles.

15. A method according to claim 14, wherein haematite, titanium dioxide, carbon black or magnetite is/are included in the mixture of monomers.

16. A method according to any of claims 9 to 15, further comprising visualizing the fingerprints using an optical method, optionally wherein the optical method is selected from, a UV search light, an optical scanner including a flat-bed optical scanner, a fluorescent scanner and a UV visible scanner.

17. A method according to any preceding claim, wherein the silica particles are microparticles having an average diameter of from about 10 to about 90µm, optionally from about 45 to about 65µm and further optionally from about 65 to about 90µm.

18. A method according to claim 15, wherein the silica particles are nanoparticles having an average diameter of about 200 to about 900nm, optionally an average diameter from about 300 to about 600nm and further optionally an average diameter of about 400 to about 500nm.

19. A method according to claim 18, wherein the nanoparticles are contacted with the area in a liquid medium.

## Patentansprüche

1. Verfahren zum Nachweis und/oder zur Identifizierung eines Fingerabdrucks oder einer anderen hydrophoben Masse oder Ablagerung, umfassend das Inkontaktbringen eines Bereiches, auf dem ein Fingerabdruck ist oder sein kann, mit einem Mittel, das hydrophobe Kieselsäurepartikel umfasst, wobei das Verfahren das Auftragen des Mittels als ein Pudermittel oder als eine Suspension, die Wasser und ein wassermischbares Lösungsmittel umfasst, auf den Bereich umfasst.

2. Verfahren nach Anspruch 1, wobei die Kieselsäurepartikel durch ein Verfahren erlangbar sind, das die gemeinsame Reaktion einer Mischung aus Silanether-Monomeren und organisch modifizierten Silanether-Monomeren mit einem Hydrolysemittel in einem einzigen Schritt umfasst.

3. Verfahren nach Anspruch 2, wobei die Kieselsäurepartikel durch ein Verfahren erlangbar sind, in dem die Silanether-Monomere TAOS-(Tetraalkoxysilan)-Monomere sind.

4. Verfahren nach Anspruch 2 oder 3, wobei die Kieselsäurepartikel durch ein Verfahren erlangbar sind, in dem das organisch modifizierte Silanether-Monomer eine arylhaltige Gruppe umfasst.

5. Verfahren nach einem der Ansprüche 2 bis 4, wobei die Kieselsäurepartikel durch ein Verfahren erlangbar sind, das die gemeinsame Reaktion einer Mischung aus TEOS-(Tetraethoxysilan)- und PTEOS-(Triethoxy(phenyl)silan)-Monomeren mit dem Hydrolysemittel in einem einzigen Schritt umfasst.

6. Verfahren nach einem der Ansprüche 2 bis 5, wobei die Kieselsäurepartikel durch ein Verfahren erlangbar sind, in dem das Hydrolysemittel eine Säure oder ein Alkali ist.

7. Verfahren nach Anspruch 6, wobei die Kieselsäurepartikel durch ein Verfahren erlangbar sind, in dem das Alkali ein Hydroxid, und gegebenenfalls Ammoniumhydroxid, ist.

8. Verfahren nach einem der Ansprüche 2 bis 7, wobei die Kieselsäurepartikel durch ein Verfahren erlangbar sind, in dem die Mischung aus Monomeren und dem Hydrolysemittel Farbstoffmoleküle und/oder farbige Partikel umfasst, die dadurch in die hydrophoben Kieselsäurepartikel eingebracht werden.

9. Verfahren nach einem der vorhergehenden Ansprüche, ferner das Visualisieren oder Abbilden der Fingerabdrücke umfassend, wobei die Kieselsäurepartikel ein Mittel zum Visualisieren oder Abbilden umfassen und das Verfahren das Visualisieren und/oder Abbilden der Partikel umfasst, nachdem die Oberfläche mit ihnen in Kontakt gebracht worden ist.

10. Verfahren nach Anspruch 9, wobei die Partikel ein Farbstoffmolekül zum Abbilden des Fingerabdrucks umfassen.

11. Verfahren nach Anspruch 10, wobei der Farbstoff ein fluoreszierender Farbstoff ist.

12. Verfahren nach Anspruch 10, wobei das Kieselsäurepartikel ein Farbstoffmolekül und/oder farbige Partikel umfasst, die aus Rhodamin B, Rhodamin 6G, Industrieruß, Titandioxid, Magnetit, Kristallviolett und Methylenblau ausgewählt sind.

13. Verfahren nach einem der vorhergehenden Ansprüche, wobei die Partikel ein magnetisches oder paramagnetisches Partikel umfassen, wodurch das Mittel mittels eines Magnetapplikators auf den Bereich aufgetragen werden kann.

14. Verfahren nach Anspruch 13, wobei die Kieselsäurepartikel durch ein Verfahren erlangbar sind, in dem die Mischung aus Monomeren und dem Hydrolysemittel das magnetische und/oder paramagnetische Partikel umfasst, das dadurch in die hydrophoben Kieselsäurepartikel eingebracht wird.

15. Verfahren nach Anspruch 14, wobei die Mischung aus Monomeren Hämatit, Titandioxid, Industrieruß oder Magnetit umfasst.

16. Verfahren nach einem der Ansprüche 9 bis 15, ferner das Visualisieren der Fingerabdrücke mittels eines optischen Verfahrens umfassend, wobei das optische Verfahren gegebenenfalls aus einem UV-Scheinwerfer, einem optischen Scanner, der einen optischen Flachbettscanner umfasst, einem Fluoreszenz-Scanner und einem UV/VIS-Scanner ausgewählt ist.

17. Verfahren nach einem der vorhergehenden Ansprüche, wobei die Kieselsäurepartikel Mikropartikel sind, die einen durchschnittlichen Durchmesser von circa 10 bis circa 90 µm, gegebenenfalls von circa 45 bis circa 65 µm und ferner gegebenenfalls von circa 65 bis circa 90 µm aufweisen.

18. Verfahren nach Anspruch 15, wobei die Kieselsäurepartikel Nanopartikel sind, die einen durchschnittlichen Durchmesser von circa 200 bis circa 900 nm, gegebenenfalls einen durchschnittlichen Durchmesser von circa 300 bis circa 600 nm und ferner gegebenenfalls einen durchschnittlichen Durchmesser von circa 400 bis circa 500 nm aufweisen.

19. Verfahren nach Anspruch 18, wobei die Nanopartikel mit dem Bereich in einem flüssigen Medium in Kontakt gebracht werden.

## Revendications

1. Procédé permettant la détection et/ou l'identification d'une empreinte digitale ou d'une autre masse ou dépôt hydrophobe comprenant la mise en contact d'une zone où se trouve ou peut se trouver une empreinte digitale avec un agent comprenant des particules de silice hydrophobes, ledit procédé comprenant l'application de l'agent sous la forme d'un agent de poudrage ou sous la forme d'une suspension qui comprend de l'eau et un solvant miscible dans l'eau sur la zone.

2. Procédé selon la revendication 1, lesdites particules de silice pouvant être obtenues par un procédé comprenant la réaction en une seule étape d'un mélange de monomères silanes éthers et de monomères silanes éthers organiquement modifiés avec un agent hydrolysant.

3. Procédé selon la revendication 2, lesdites particules de silice pouvant être obtenues par un procédé dans lequel les monomères silanes éthers sont des monomères de TAOS (tétraalcoxysilane).

4. Procédé selon la revendication 2 ou la revendication 3, lesdites particules de silice pouvant être obtenues par un procédé dans lequel le monomère silane éther organiquement modifié comprend un groupe contenant un aryle.

5. Procédé selon l'une quelconque des revendications 2 à 4, lesdites particules de silice pouvant être obtenues par un procédé qui comprend la réaction en une seule étape d'un mélange de monomères de TEOS (tétraéthoxysilane) et de PTEOS (phényltriéthoxysilane) avec l'agent hydrolysant.

6. Procédé selon l'une quelconque des revendications 2 à 5, lesdites particules pouvant être obtenues par un procédé dans lequel l'agent hydrolysant est un acide ou un alcali.

7. Procédé selon la revendication 6, lesdites particules de silice pouvant être obtenues par un procédé dans lequel l'alcali est un hydroxyde et est éventuellement de l'hydroxyde d'ammonium.

8. Procédé selon l'une quelconque des revendications 2 à 7, lesdites particules de silice pouvant être obtenues par un procédé dans lequel des molécules de colorant et/ou des particules colorées sont incluses dans le mélange des monomères et de l'agent hydrolysant et sont ainsi incorporées dans les particules de silice hydrophobes.

9. Procédé selon l'une quelconque des revendications précédentes, comprenant en outre la visualisation ou l'imagerie des empreintes digitales, lesdites particules de silice comprenant un agent de de visualisation ou d'imagerie et ledit procédé comprenant la visualisation et/ou l'imagerie des particules une fois que la surface a été en contact avec elles.

10. Procédé selon la revendication 9, lesdites particules comprenant une molécule de colorant permettant l'imagerie de l'empreinte digitale.

11. Procédé selon la revendication 10, ledit colorant étant un colorant fluorescent.

12. Procédé selon la revendication 10, ladite particule de silice comprenant une molécule de colorant et/ou des particules colorées, qui sont choisies parmi la rhodamine B, la rhodamine 6G, le noir de carbone, le dioxyde de titane, la magnétite, le cristal violet et le bleu de méthylène.

13. Procédé selon l'une quelconque des revendications précédentes, lesdites particules comprenant une particule magnétique ou paramagnétique qui permet l'application de l'agent sur la zone à l'aide d'un applicateur magnétique.

14. Procédé selon la revendication 13, lesdites particules de silice pouvant être obtenues par un procédé dans lequel les particules magnétiques et/ou paramagnétiques sont incluses dans le mélange des monomères et de l'agent hydrolysant et sont ainsi incorporées dans les particules de silice hydrophobes.

15. Procédé selon la revendication 14, de l'hématite, du dioxyde de titane, du noir de carbone ou de la magnétite étant inclus dans le mélange de monomères.

16. Procédé selon l'une quelconque des revendications 9 à 15, comprenant en outre la visualisation des empreintes digitales à l'aide d'un procédé optique, éventuellement ledit procédé optique étant choisi parmi une lampe de recherche par UV, un scanner optique dont un scanner optique à champ plat, un scanner par fluorescence et un scanner UV-visible.

17. Procédé selon l'une quelconque des revendications précédentes, lesdites particules de silice étant des microparticules ayant un diamètre moyen allant d'environ 10 à environ 90 µm, éventuellement d'environ 45 à environ 65 µm et en outre éventuellement d'environ 65 à environ 90 µm.

18. Procédé selon la revendication 15, lesdites particules de silice étant des nanoparticules ayant un diamètre moyen d'environ 200 à environ 900 nm, éventuellement un diamètre moyen d'environ 300 à environ 600 nm et en outre éventuellement un diamètre moyen d'environ 400 à environ 500 nm.

19. Procédé selon la revendication 18, lesdites nanoparticules étant mises en contact avec la zone dans un milieu liquide.
